Europäisches Patentamt

European Patent Office

Office européen des brevets

(11) Veröffentlichungsnummer: **0 297 523**
**A2**

## (12) EUROPÄISCHE PATENTANMELDUNG

(21) Anmeldenummer: 88110341.0

(22) Anmeldetag: 29.06.88

(51) Int. Cl.⁴: **C12P 1/06 , A61K 35/66 ,**
**//(C12P1/06,C12R1:465)**

Der Anmelder hat eine Erklärung nach Regel 28 (4) EPÜ (Herausgabe einer Probe nur an einen Sachverständigen) eingereicht. Eingangsnummern der Hinterlegungen: DSM 3816, DSM 4137.

(30) Priorität: 01.07.87 DE 3721722

(43) Veröffentlichungstag der Anmeldung:
04.01.89 Patentblatt 89/01

(84) Benannte Vertragsstaaten:
BE CH DE FR GB IT LI NL

(71) Anmelder: **HOECHST AKTIENGESELLSCHAFT**
**Postfach 80 03 20**
**D-6230 Frankfurt am Main 80(DE)**

(72) Erfinder: **Voelskow, Hartmut Dr.**
**Akazienstrasse 22**
**D-6234 Hattersheim am Main(DE)**
Erfinder: **Grabley, Susanne, Dr.**
**Hölderlinstrasse 7**
**D-6240 Königstein/Taunus(DE)**
Erfinder: **Hammann, Peter, Dr.**
**Mörikestrasse 6**
**D-6233 Kelkheim/Taunus(DE)**
Erfinder: **Düwel, Dieter Dr.**
**Frankfurter Strasse 39**
**D-6238 Hofheim am Taunus(DE)**
Erfinder: **Seibert, Gerhard Prof. Dr.**
**Gläserweg 21**
**D-6100 Darmstadt(DE)**
Erfinder: **Giani, Carlo Dr.**
**Hedderichstrasse 69**
**D-6000 Frankfurt am Main 70(DE)**
Erfinder: **Kretzschmar, Gerhard**
**Falkenstrasse 71**
**D-6232 Bad Soden am Taunus(DE)**
Erfinder: **Zeeck, Axel Prof.Dr.**
**Brüder-Grimm-Allee 22**
**D-3400 Göttingen(DE)**
Erfinder: **Kricke-Helling, Pia Dr.**
**Rauschenwasser 27**
**D-3406 Bovenden(DE)**

(54) Verfahren zur Herstellung von Elaiophylin und dessen Verwendung.

(57) Streptomyces parvullus, DSM 3816, und Streptomyces spec. DSM 4137 produzieren in aerober Fermentation Elaiophylin, das sich durch eine anthelminthische Wirkung auszeichnet.

## Verfahren zur Herstellung von Elaiophylin und dessen Verwendung

Elaiophylin wurde vor über 25 Jahren zuerst von Arcamone et al. [J. Microbiol. 7, 207, (1959)] isoliert und später von Arai unter dem Namen Azalomycin B beschrieben. Kaiser et al. [Helv. Chim. Acta, 64, 407, (1981)] isolierten Elaiophylin zusammen mit Nigericin und den Niphithricinen A und B aus Kulturen von Streptomyces violaceoniger.

Im Journal of Antibiotics, Vol. 34 (1981), 1107 wird die Fermentation eines Isolates von Streptomyces violaceoniger (TÜ 905) beschrieben. Bei diesem Verfahren werden ca. 100 mg Elaiophylin pro Liter Kulturbrühe erhalten.

Es wurde bereits vorgeschlagen (P 37 00 325.9), den Stamm Streptomyces parvullus, der bei der Deutschen Sammlung von Mikroorganismen unter der Nummer DSM 3816 hinterlegt wurde, zur Synthese des Antibiotikums Amycin, welches antifungische Wirkung entfaltet, zu verwenden.

In der DE-A 32.48 280 wird ein Verfahren zur Herstellung von Salbomycin beschrieben. Salbomycin entspricht in seiner chemischen Struktur Elaiophylin. Das Verfahren zur Herstellung von Salbomycin beruht auf der Kultivierung von Streptomyces albus, insbesondere Streptomyces albus ATCC 21838 (DSM 2566). Die Salbomycinausbeuten betragen 6 - 140 mg/l Kulturbrühe.

Es wurde nun überraschend gefunden, daß sich der Stamm Streptomyces parvullus, DSM 3816, sowie seine Varianten und/oder Mutanten auch zur Herstellung von Elaiophylin eignen. Ebenso eignet sich der Stamm Streptomyces species, DSM 4137, der ebenfalls bei der Deutschen Sammlung von Mikroorganismen hinterlegt wurde, hervorragend zur Herstellung von Elaiophylin, wobei die Ausbeuten an Elaiophylin weit über den Werten, die in der Literatur für ähnliche Streptomyces species angegeben sind, liegen. Weiterhin zeigten Untersuchungen, daß sich Elaiophylin besonders gut als Anthelminthikum eignet.

Die Erfindung betrifft somit:

1. Ein Verfahren zur Herstellung von Elaiophylin, das dadurch gekennzeichnet ist, daß
   Streptomyces parvullus, DSM 3816, sowie seine Varianten und Mutanten und/oder
   Streptomyces species, DSM 4137 sowie seine Varianten und Mutanten
in einem Nährmedium kultiviert werden, bis sich Elaiophylin in der Kultur anhäuft und daß gegebenenfalls anschließend das gebildete Elaiophylin isoliert wird.

2. Die Verwendung von Elaiophylin als Anthelminthikum.

Im folgenden wird die Erfindung detailliert, insbesondere in ihren bevorzugten Ausführungsformen, beschrieben. Ferner wird die Erfindung in den Patentansprüchen definiert.

In einer Nährlösung, die eine Kohlenstoffquelle und eine Stickstoffquelle sowie die üblichen anorganischen Salze enthält, produzieren sowohl Streptomyces parvullus, DSM 3816 als auch Streptomyces spec., DSM 4137, Elaiophylin. Anstelle der Stämme DSM 3816 oder DSM 4137 können natürlich auch deren Mutanten und/oder Varianten eingesetzt werden, soweit sie Elaiophylin synthetisieren. Solche Mutanten können in an sich bekannter Weise durch physikalische Mittel, beispielsweise durch Bestrahlung mit Ultraviolett- oder Röntgenstrahlen, oder durch chemische Mutagene, wie Ethylmethansulfonat (EMS) oder 2-Hydroxy-4-methoxybenzophenon (MOB), erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Rohstoffe, wie Malzextrakt, Melasse oder Maltodextrine. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate von Eisen, Zink und Mangan oder von Alkali- oder Erdalkalimetallen enthalten.

Die Bildung des Elaiophylin verläuft besonders gut in einer Nährlösung, die, insbesondere für die Kultivierung von DSM 3816, Sojamehl und Mannit und, im Falle der Kultivierung von DSM 4137, Melasse und gegebenenfalls Maltodextrine enthält. Dabei werden diese Nährstoffe (Sojamehl, Mannit, Melasse, Maltodextrine) jeweils in Konzentrationen von 0,5 - 5 %, bevorzugt 1 - 3 %, bezogen auf das Gewicht der gesamten Nährlösung, eingesetzt.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35°C, vorzugsweise bei etwa 25 bis 30°C, insbesondere bei 28 bis 30°C erfolgen. Der pH-Bereich sollte zwischen 6,0 und 8,5 liegen, vorteilhaft zwischen 6,5 und 8,0.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar oder Glycerin-Arginin-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden. Elaiophylin ist sowohl im Mycel als auch im Kulturfiltrat enthalten, insbesondere im Mycel.

Nach dem erfindungsgemäßen Verfahren können Elaiophylin-Konzentrationen von bis zu 2 g/l Kulturbrühe erreicht werden. Damit wird die Ausbeute an Elaiophylin sowie die Wirtschaftlichkeit des Verfahrens im Vergleich zu bisher bekannten Verfahren wesentlich erhöht.

Zum Testen der Elaiophylin-Konzentration in der Kultur kann die Dünnschichtchromatographie, beispielsweise auf Kieselgel mit Chloroform/Methanol als Laufmittel verwendet werden, wobei die Menge des gebildeten Elaiophylin zweckmäßig mit einer Eichlösung verglichen wird.

Wenn die Isolierung von Elaiophylin notwendig ist, werden Kulturbrühe und Mycel, bevorzugt nur das Mycel, mit organischen Lösungsmitteln, wie Chloroform oder Essigester, bevorzugt Essigester, extrahiert.

Die Reinisolierung erfolgt durch Kristallisation aus dem organischen Lösungsmittel, bevorzugt aus Essigester. Diese vereinfachte Reinisolierung trägt ebenso wie die erhöhte Ausbeute zur Erhöhung der Wirtschaftlichkeit des Verfahrens bei.

Elaiophylin und dessen Derivate zeigen anthelminthische Wirkung, insbesondere gegen Haemonchus, Trichostrongylus, Ostertagia, Cooperia, Chabertia, Strongyloides, Oesophagostomum, Hyostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden.

Elaiophylin kann grundsätzlich als solches in Substanz verabreicht werden. Bevorzugt ist die Verwendung in Mischung mit geeignetem Trägermaterial. Als Trägermaterial können die üblichen Futtermittelmischungen verwendet werden.

In den sich anschließenden Beispielen wird die Erfindung weiter erläutert. Prozentangaben beziehen sich auf das Gewicht und Mischungsverhältnisse bei Flüssigkeiten beziehen sich auf das Volumen, wenn keine anderen Angaben gemacht wurden.

Beispiele

1.

a) Herstellung einer Sporensuspension von Streptomyces parvullus DSM 3816 oder Streptomyces spec. DSM 4137

Zusammensetzung des Nährmediums:

12,5 g Glycerin
1,0 g Arginin
1,0 g NaCl
1,0 g $K_2HPO_4$
0,5 g $MgSO_4.7H_2O$
15,0 g Agar
2,5 ml Spurenelementlösung
1 l $H_2O$ dest.

Zusammensetzung der Spurenelementlösung:

30 g $CaCl_2.2H_2O$
1,0 g Eisen-III-citrat
0,2 g $MnSO_4$
0,1 g $ZnCl_2$
0,025 g $CuSO_4.5H_2O$
0,02 g $Na_2B_4O_7.10H_2O$
0,004 g $CaCl_2$
0,01 g $Na_2MoO_4.2H_2O$
1 l $H_2O$ dest.

Schrägröhrchen mit obengenanntem Nährmedium werden mit DSM 3816 bzw. DSM 4137 beimpft und 8 Tage bei 30˚C bebrütet. Die Sporen der Röhrchen werden mit 3 ml einer Lösung aus 0,9 % NaCl und 0,1 % Tween[R] 80 abgeschwemmt und bis zur Beimpfung bei 4˚C aufbewahrt.

b) Herstellung einer Vorkultur von DSM 3816 bzw. DSM 4137 im Erlenmeyerkolben

Erlenmeyerkolben von 300 ml Fassungsvermögen werden mit je 100 ml Nährlösung (40 g Glucose, 30 g Sojamehl, 2,5 g NaCl, 2,5 g $CaCO_3$, in 1 l dest. Wasser, pH 7,5 vor der Sterilisation) beschickt und mit je 1,5 ml der nach 1a) hergestellten, möglichst frischen Sporensuspension angeimpft. Es wird auf einer Schüttelmaschine (180 UPM) bei 30˚C 72 h inkubiert.

c) I Herstellung einer Hauptkultur von DSM 3816

Ein 300 ml Erlenmeyerkolben mit 100 ml Nährlösung (20 g Sojamehl, 30 g Mannit, in 1 l dest. Wasser, pH 7,5 vor der Sterilisation) wird mit 3 ml der DSM 3816-Vorkultur gemäß 1b beimpft und bei 30˚C auf der Schüttelmaschine (180 UPM) inkubiert. Das Produktionsmaximum wird nach ca. 48 h bis 150 h erreicht.
Die Ausbeuten liegen bei 300 bis 500 mg Elaiophylin pro l.

II. Herstellung einer Hauptkultur von DSM 4137

Ein 300 ml Erlenmeyerkolben mit 100 ml Nährlösung (10 g Sojamehl, 20 g Mannit, 10 g Melasse, in 1 l dest. Wasser, pH 7,5 vor der Sterilisation) wird mit 3 ml der DSM 4137-Vorkultur gemäß 1b beimpft und bei 30˚C auf der Schüttelmaschine (180 UPM) inkubiert. Das Produktionsmaximum wird nach ca. 48 h bis 150 h erreicht.
Die Ausbeuten liegen bei 1200 bis 2000 mg Elaiophylin pro l.

d) Kultivierung von DSM 3816 bzw. 4137 im Fermenter

Ein Fermenter von 13 l Fassungsvermögen wird unter folgenden Bedingungen betrieben:
Bei 30˚C Inkubationstemperatur und 500 UPM des Rührers werden 7 l Luft pro Minute in die Kulturflüssigkeit (Medium wie in Beispiel 1c) eingeleitet. Der Fermenter wird mit 200 ml der Vorkultur (siehe 1b) beimpft. Über den Fermentationszeitraum wird durch Zudosierung von NaOH der pH-Wert auf einem konstanten Wert von pH 6.9 gehalten. Das Produktionsoptimum wird nach ca. 48 bis 150 h erreicht. Die Ausbeuten liegen bei ca. 300 bis 400 mg Elaiophylin/l (DSM 3816) bzw. 1200 bis 1300 mg Elaiophylin/l (DSM 4137).

2. Aufarbeitung von 20 l Kulturbrühe

Die Kulturbrühe wird filtriert. Das Mycel wird mit Essigester (3x500 ml) extrahiert und anschließend auf 200 ml reduziert, wobei das Elaiophylin auskristallisiert. Man erhält 7,0 g Elaiophylin (DSM 3816) bzw: 25g Elaiophylin (DSM 4137).

Das Kulturfiltrat wird mit 3 x 20 l Essigester extrahiert. Die organische Phase wird im Vakuum auf 100 ml eingeengt, wobei 0,9 g (DSM 3816) bzw. 1,1 g (DSM 4137) Elaiophylin auskristallisiert.

Fp. Elaiophylin: 198 - 200°C

3. Anthelminthische Wirkung von Elaiophylin

Die anthelminthische Wirkung vom Elaiophylin wurde an Lämmern mit 30 - 40 kg Körpergewicht untersucht. Dazu wurden die Lämmer artifiziell mit Infektionsstadien von Labmagen- (Haemonchus contortus) und Dünndarm-(Trichostrongylus colubriformis) Nematoden infiziert. Nach Abschluß der Entwicklungszeit (Präpatenzperiode) der Nematoden erfolgte die Applikation von Elaiophylin in Dosierungen von 2,5, 5,0 und 10,0 mg/kg (p.o.) bzw. 2,5 mg/kg (subcutan).

Durch koproskopische Untersuchungen vor und bis zu 14 Tagen nach der Applikation des Elaiophylins und anschließende Sektion mit helminthologischer Aufarbeitung wurde die prozentuale Reduktion der Schaf-Nematoden ermittelt (s. Tab. 1).

Tabelle 1

| Elaiophylingabe | proz. Reduktion der Nematoden | |
|---|---|---|
| | H. contortus | T. colubriformis |
| 2,5 mg s.c. | 46,5 % | 35,1 % |
| 2,5 mg p.o. | 44,6 % | 28,4 % |
| 5,0 mg p.o. | 89,3 % | 73,8 % |
| 10,0 mg p.o. | 85,0 % | 73,8 % |

**Ansprüche**

1. Verfahren zur Herstellung von Elaiophylin, dadurch gekennzeichnet, daß entweder
    Streptomyces parvullus, DSM 3816, sowie seine Varianten und Mutanten und/oder
    Streptomyces species, DSM 4137, sowie seine Varianten und Mutanten
in einem Nährmedium kultiviert werden, bis sich Elaiophylin in der Kultur anhäuft und daß gegebenenfalls anschließend das gebildete Elaiophylin isoliert wird.

2. Verfahren nach Anspruch 1, dadurch gekennzeichnet, daß der pH-wert während der Fermentation zwischen 6,0 und 8,5 gehalten wird.

3. Verfahren nach Anspruch 1, dadurch gekennzeichnet, daß die Fermentation in einem Temperaturbereich von 18 bis 35°C durchgeführt wird.

4. Verfahren nach Anspruch 1, dadurch gekennzeichnet, daß man in mehreren Stufen kultiviert.

5. Verfahren nach Anspruch 1, dadurch gekennzeichnet, daß DSM 3816 in einer Nährlösung fermentiert wird, die Sojamehl und Mannit enthält.

6. Verfahren nach Anspruch 1, dadurch gekennzeichnet, daß DSM 4137 in einer Nährlösung fermentiert wird, die Melasse und/oder Maltodextrin enthält.

7. Verfahren nach Anspruch 1, dadurch gekennzeichnet, daß die Isolierung des Elaiophylins aus dem Mycel erfolgt.

8. Verfahren nach Anspruch 1 oder 7, dadurch gekennzeichnet, daß die Isolierung des Elaiophylins durch Extraktion mit Essigester erfolgt.

9. Verwendung von Elaiophylin zur Herstellung von Anthelminthika.

10. Verwendung von Elaiophylin zur Bekämpfung von Magen-Darm-Strongyliden und Lungenwürmern.

11. Tierarzneimittel, dadurch gekennzeichnet, daß es Elaiophylin enthält oder daraus besteht.

Patentanspruch für folgende Vertragsstaaten : ES, GR

1. Verfahren zur Herstellung von Elaiophylin, dadurch gekennzeichnet, daß entweder
(a) Streptomyces parvullus, DSM 3816, und/oder seine Varianten und/oder Mutanten oder
(b) Streptomyces species, DSM 4137, und/oder seine Varianten und/oder Mutanten
in einem Nährmedium kultiviert werden, bis sich Elaiophylin in der Kultur anhäuft und daß man gegebenenfalls anschließend das gebildete Elaiophylin isoliert.

2. Verfahren nach Anspruch 1, dadurch gekennzeichnet, daß der pH-wert während der Fermentation zwischen 5,5 und 8,5 gehalten wird.

3. Verfahren nach Anspruch 1, dadurch gekennzeichnet, daß die Fermentation in einem Temperaturbereich von 18 bis 35 °C durchgeführt wird.

4. Verfahren nach Anspruch 1, dadurch gekennzeichnet, daß man in mehreren Stufen kultiviert.

5. Verfahren nach Anspruch 1, dadurch gekennzeichnet, daß DSM 3816 in einer Nährlösung fermentiert wird, die Sojamehl und Mannit enthält.

6. Verfahren nach Anspruch 1, dadurch gekennzeichnet, daß DSM 4137 in einer Nährlösung fermentiert wird, die Melasse und/oder Maltodextrin enthält.

7. Verfahren nach Anspruch 1, dadurch gekennzeichnet, daß die Isolierung des Elaiophylins aus dem Mycel erfolgt.

8. Verfahren nach Anspruch 1 oder 7, dadurch gekennzeichnet, daß die isolierunq des Elaiophylins durch Extraktion mit Essigester erfolgt.

9. Verwendung von Elaiophylin zur Herstellung von Anthelminthika.

10. Verwendung von Elaiophylin zur Bekämpfung von Magen-Darm-Strongyliden und Lungenwürmern.